# EUROPÄISCHE PATENTSCHRIFT

(11) **EP 0 648 465 B1**
(45) Veröffentlichungstag und Bekanntmachung des Hinweises auf die Patenterteilung: **23.06.1999**
(21) Anmeldenummer: 94202988.5
(22) Anmeldetag: 14.10.1994
(51) Int. Cl.: A61B 6/00

(54) **Röntgenuntersuchungsgerät**
X-ray examination apparatus
Appareil d'examen à rayons x

(30) Priorität: 16.10.1993 DE 4335306
(43) Veröffentlichungstag der Anmeldung: 19.04.1995
(73) Patentinhaber: Philips Patentverwaltung GmbH, 22335 Hamburg (DE); Koninklijke Philips Electronics N.V., 5621 BA Eindhoven (NL)
(72) Erfinder: Kayser, Harald, Dr.-Ing., D-22335 Hamburg (DE)
(74) Vertreter: Hartmann, Heinrich, Dipl.-Ing.

(56) Entgegenhaltungen:
- EP-A- 0 061 390
- EP-A- 0 297 355
- DE-A- 2 932 722
- DE-C- 836 698
- US-A- 4 602 378

## Beschreibung

Die Erfindung betrifft ein Röntgenuntersuchungsgerät gemäß dem Oberbegriff des Anspruchs 1. Ein solches Röntgenuntersuchungsgerät ist aus der EP-OS 160 340 oder aus der US-A-4 602 378 bekannt. Mit einem Röntgenuntersuchungsgerät dieser Art kann der Untersucher eine Röntgendurchleuchtung eines Patienten durchführen und Röntgenaufnahmen anfertigen. Bei diesen Röntgenaufnahmen befindet sich - bei horizontalem Patientenlagerungstisch - der Röntgenstrahler unterhalb des Patientenlagerungstisches und die Röntgenaufnahmeneinrichtung in dem Zielgerät oberhalb dieses Tisches. Man kann derartige Röntgenuntersuchungsgeräte aber auch dazu benutzen, mit einer zweiten, oberhalb des Patientenlagerungstisches befindlichen Röntgenröhre Röntgenaufnahmen auf einen Film anzufertigen, der sich in einer Laufrastereinrichtung befindet, die im Röntgenuntersuchungsgerät unterhalb der Tischplatte angeordnet ist. Diese Röntgenaufnahmen werden auch als "Obertisch-Röntgenaufnahmen" bezeichnet. Dadurch ergibt sich ein weiterer Aufnahme-Arbeitsplatz, ohne daß ein zusätzlicher Röntgengenerator und ein Aufnahmetisch erforderlich sind.

Aufgabe der vorliegenden Erfindung ist es, ein Röntgenuntersuchungsgerät der eingangs genannten Art so auszugestalten, daß der Aufwand für die Obertischtechnik noch weiter reduziert wird. Diese Aufgabe wird erfindungsgemäß durch die im Anspruch 1 angegebenen Maßnahmen gelöst.

Es sei an dieser Stelle erwähnt, daß aus der DE-C-836 698 bereits ein Röntgenuntersuchungsgerät mit einem horizontalen Patientenlagerungstisch bekannt ist, bei dem der Röntgenstrahler und das Zielgerät fest miteinander verbunden und um eine horizontale Achse um 90° schwenkbar sind. Diese horizontale Achse kann in einer Tragsäule in vertikaler Richtung verschoben werden.

Bei einem Röntgenuntersuchungsgerät nach der Erfindung kann der Röntgenstrahler aus seiner Normalstellung, in der er auf das Röntgenzielgerät zentriert ist, um die zweite Achse nach oben geschwenkt werden, so daß damit Obertisch-Röntgenaufnahmen in Verbindung mit einer unterhalb der Tischplatte befindlichen Laufrastreinrichtung ausgeführt werden können; das Röntgenzielgerät bleibt dabei oberhalb des Patientenlagerungstisches.

Mit dem erfindungsgemäßen Röntgenuntersuchungsgerät sind also Obertisch-Röntgenaufnahmen ohne einen zweiten Röntgenstrahler und die dafür erforderlichen Komponenten, wie Stativ, Hochspannungskabel, Tiefenblende usw. möglich. Grundsätzlich könnte die zweite Achse auch parallel zur Längsrichtung der Tischplatte verlaufen; vorzugsweise verläuft sie aber parallel zur ersten Achse.

Bei der bevorzugten Weiterbildung der Erfindung nach Anspruch 2 befinden sich - wie bei Röntgenuntersuchungsgeräten der eingangs genannten Art üblich - der Fokus des Röntgenstrahlers und die Mitte des Röntgenzielgerätes in einer zur Längsrichtung der Tischplatte senkrechten Ebene, jedoch befindet sich die zweite Achse außerhalb der Ebene. Deshalb befindet sich der Röntgenstrahler nach einer Schwenkung um 180° oberhalb der Tischplatte und ist gegenüber dem Röntgenzielgerät in Längsrichtung versetzt. Dadurch ist eine Kollision zwischen Röntgenstrahler und Zielgerät ausgeschlossen und das Zielgerät befindet sich außerhalb des Strahlengangs der oberhalb der Tischplatte befindlichen Röntgenröhre.

Durch die Ausgestaltung der Erfindung nach Anspruch 3 wird erreicht, daß die Laufrastereinrichtung in Längsrichtung der Tischplatte einen definierten Abstand vom Röntgenzielgerät hat. Wenn das gleiche für den Röntgenstrahler gilt, sind der Röntgenstrahler in der Obertisch-Position und die Laufrastereinrichtung automatisch aufeinander ausgerichtet. Die Ausgestaltung der Erfindung nach Anspruch 4 macht mit geringem zusätzlichen Aufwand - die Mittel für die motorische Verschiebung sind für andere Zwecke meist ohnehin schon vorhanden - eine lineare Tomographie möglich.

Die Erfindung wird nachstehend anhand der Zeichnung näher erläutert. Es zeigen
- Fig. 1: das erfindungsgemäße Röntgenuntersuchungsgerät von der Kopfseite aus gesehen und
- Fig. 2: das gleiche Gerät in einer seitlichen Darstellung.

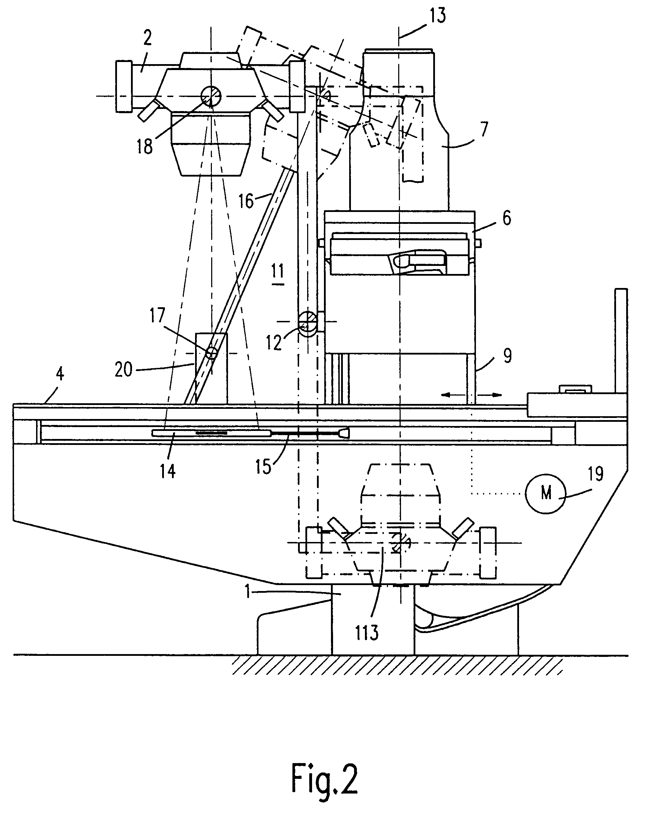

In den Figuren ist mit 1 die Basis des Röntgenuntersuchungsgeräts bezeichnet, die das Gerät trägt und es mit dem Fußboden verbindet. An der Basis 1 ist ein Patientenlagerungstisch 22 um eine horizontale Achse 3 schwenkbar gelagert. Der Patientenlagerungstisch ist mit einer motorisch angetriebenen Tischplatte 4 versehen, d.h. einer Tischplatte, die in ihrer Ebene verschoben werden kann. In der aus Fig. 2 ersichtlichen horizontalen Stellung der Tischplatte befindet sich unterhalb der Tischplatte ein Röntgenstrahler 2 (Fig. 2). Oberhalb der Tischplatte befindet sich ein Röntgenzielgerät 6, das in der Regel einen Bildverstärker 7 umfaßt.

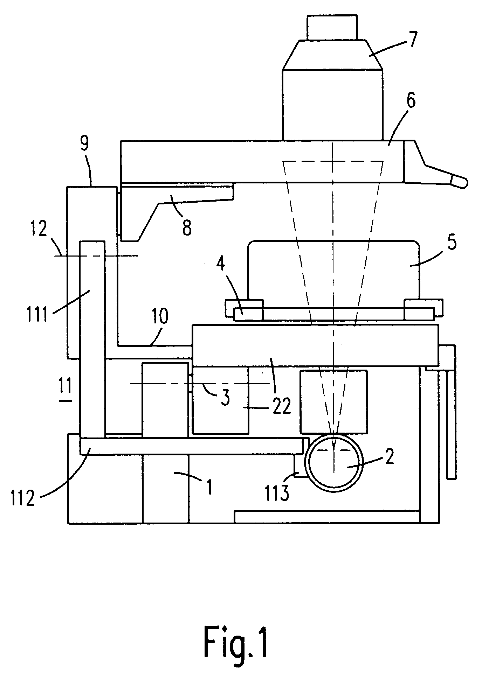

In der in Fig. 1 dargestellten Stellung des Röntgenstrahlers 2 ist dieser auf das Zielgerät 6 ausgerichtet, d.h. der Fokus des Röntgenstrahlers, von dem die Röntgenstrahlen ausgehen und die Mitte des Eingangsschirms des Röntgenbildverstärkers liegen auf einer Geraden, die senkrecht zur Tischplatte 4 verläuft. Diese Ausrichtung bleibt auch bei einer Schwenkung um die Achse 3 erhalten und (dabei werden Röntgenstrahler 2 und Zielgerät 6 mitgeschwenkt) und auch bei einer Verschiebung des Röntgenzielgeräts in Längsrichtung der Tischplatte oder in Querrichtung (parallel zur Schwenkachse 3).

Das Zielgerät 6 ist darüberhinaus in Kompressionsrichtung bewegbar, d.h. senkrecht zur Tischplatte 4. Zu diesem Zweck ist ein Kompressionswagen 8 vorgesehen, der an einem sogenannten Turm 9 in Kompressionsrichtung verschiebbar geführt ist. Der Turm 9 seinerseits wird von einer Transporteinrichtung in Form eines Wagens 10 getragen, der unterhalb der Tischplatte 4 in Längs- und in Querrichtung verschiebbar ist. Wird das Zielgerät 6 in Längsrichtung oder in Querrichtung verschoben, dann verschiebt sich damit auch der Wagen 10 sowie der damit gekoppelte Röntgenstrahler 2, so daß Röntgenstrahler 2 und Zielgerät 6 bei einer solchen Verschiebung aufeinander zentriert bleiben. Insoweit als bisher beschrieben, ist das Röntgenuntersuchungsgrät schon aus der eingangs erwähnten EP-OS 160 340 bekannt.

Erfindungsgemäß ist der Röntgenstrahler 2 nicht starr mit dem Turm 9 bzw. dem Wagen 10 verbunden, sondern an einem Träger 11 befestigt, der um eine vorzugsweise oberhalb der Tischplatte 4 parallel zur Achse 3 verlaufende, mit dem Turm 9 (oder dem Wagen 10) verbundene Achse 12 schwenkbar ist. Der Träger umfaßt einen mit seinem einen Ende um die Achse 12 schwenkbaren Arm 111, an dessem anderen Ende ein in Querrichtung verlaufender Arm 112 befestigt ist, der seinerseits einen senkrecht zu den beiden Armen 111, 112 verlaufenden dritten Arm 113 trägt, mit dem der Röntgenstrahler 2 verbunden ist. Diese drei Arme des Trägers 11 stellen sicher, daß der Röntgenstrahler 2 und der Bildverstärker 7 am Röntgenzielgerät 6 aufeinander zentriert sind, obwohl die Schwenkachse 12 nicht durch die zur Tischplatte 4 senkrechte Ebene 13 verläuft, die den Fokus des Röntgenstrahlers und den Mittelpunkt des Verstärkereingangsschirms enthält. Wie Fig. 2 deutlich zeigt, ist die Schwenkachse 12 in bezug auf die Ebene 13 in Richtung auf das Kopfende versetzt.

Um das Röntgenuntersuchungsgerät für eine Obertisch-Röntgenaufnahme vorzubereiten, muß Röntgenstrahler 2 um 180° geschwenkt werden. Hierzu wird das Röntgenzielgerät (und mit ihm der Wagen 10 und der Röntgenstrahler 2) zum Kopfende (in Fig. 2 ist das die linke Seite) verfahren. In dieser Endstellung wird eine nicht näher dargestellte Arretierung, die den Träger 11 bezüglich der Schwenkachse 12 fixiert, gelöst und der Träger um 180° um die Achse 12 nach oben geschwenkt und in dieser Position, in der er in Fig. 2 mit ausgezogenen Linien dargestellt ist, erneut arretiert. Diese Schwenkbewegung wird vorzugsweise durch geeignete Mittel, wie z.B. eine Feder, eine Gasdruckfeder oder dergl. in der Weise unterstützt, daß das vom Röntgenstrahler bezüglich der Achse 12 erzeugte Moment kompensiert wird. In der dann erreichten und in Fig. 2 in ausgezogenen Linien dargestellten Obertisch-Position des Röntgenstrahlers ist der Abstand des Brennflecks des Röntgenstrahlers 2 von der Ebene 13 doppelt so groß wie der Abstand der Schwenkachse 12 von dieser Ebene.

Es können dann Obertischaufnahmen auf einem Film angefertigt werden, der sich in einer Laufrastereinrichtung 14 befindet. Die Laufrastereinrichtung ist unterhalb der Tischplatte 4 angeordnet und parallel dazu verschiebbar. Die Ausrichtung des Röntgenstrahlers auf die Laufrastereinrichtung 14 bzw. im darin befindlichen Film kann in üblicher Weise mit Hilfe eines Lichtvisiers erfolgen, das in der mit dem Röntgenstrahler 2 verbundenen Tiefenblende vorhanden ist. Das Ausrichten auf den Röntgenstrahler 2 kann aber bei Röntgenaufnahmen mit einem zur Tischplatte senkrechten Strahlengang entfallen, wenn die Laufrastereinrichtung mit dem Turm 9 über eine Koppelvorrichtung 15 verbunden wird, durch die Laufrastereinrichtung mit dem Turm 9 mitbewegt wird.

In der Obertisch-Position des Röntgenstrahlers 2 können damit auch Schichtaufnahmen mit linearer Verwischungsbewegung angefertigt werden. Zu diesem Zweck ist in an sich bekannter Weise eine Koppelstange 16 vorgesehen, die um eine bezüglich der Tischplatte 4 ortsfeste, zu den Achsen 3 und 12 parallele Achse 17 schwenkbar ist. Die Koppelstange 16 ist mit ihren beiden Enden mit dem Röntgenstrahler 2 bzw mit einer Filmkassette verbunden, so daß diese bei einer Schwenkung der Koppelstange um die Achse 17 gegensinnig zueinander bewegt werden. Alle Teile des Patienten, die sich außerhalb einer die Achse 17 enthaltenden, zur Tischplatte 4 parallelen Ebene befinden, werden verwischt dargestellt, und nur von der erwähnten Ebene (Schicht) gibt es eine scharfe Abbildung.

Damit in diesen Fällen der Röntgenstrahler auf die in der entgegengesetzte Richtung bewegte Filmkassette ausgerichtet bleibt, muß er um eine Achse 18 schwenkbar sein, die durch den Fokus des Röntgenstrahlers verläuft. Fig. 2 zeigt mit strichpunktierten Linien den Röntgenstrahler in einer für eine Schichtaufnahme erforderlichen Obertisch-Position. Zum Verschieben des Röntgenstrahlers während der Schichtaufnahme kann ein schematisch angedeuteter Motorantrieb 19 (Fig. 2) vorgesehen sein, der auf den Turm 9 bzw. den Wagen 10 einwirkt und diesen in Längsrichtung verschiebt. Ein solcher Motorantrieb ist bei den meisten Röntgenuntersuchungsgeräten dieser Art zur Unterstützung der Verschiebung von Röntgenzielgerät und Röntgenstrahler ohnehin vorhanden. - Es ist aber auch möglich, die gegensinnige Bewegung von Röntgenstrahler 2 und Filmkassette dadurch zu erzeugen, daß die Tischplatte angetrieben wird, wenn das Halteteil 20, in dem die Achse 17 der Koppelstange 16 gelagert ist, in diesem Fall mit der Tischplatte verbunden wird. Bei Geräten dieser Art ist ohnehin in der Regel ein Antrieb für die Tischplatte 4 vorgesehen. Allerdings wird dadurch auch während der Schichtaufnahme der auf der Tischplatte 4 befindliche Patient mitbewegt. Dies kann zu Bewegungsunschärfen führen.

Wenn der Röntgenstrahler sich auf der gleichen Seite der Tischplatte befindet wie das Röntgenzielgerät 6, sind auch noch weitere Untersuchungstechniken möglich. Beispielsweise kann das Untersuchungsgerät um die Achse 3 um 90° im Uhrzeigersinn geschwenkt werden. Wenn dann auch noch der Strahler 2 um 180° gedreht wird, können Röntgenaufnahmen mit horizontal (von der Tischplatte weg) verlaufendem Strahlengang angefertigt werden, beispielsweise auf einer an einer Wand des Untersuchungsraums befestigte Laufrastereinrichtung. - Darüberhinaus sind auch noch andere Aufnahmetechniken möglich, beispielsweise bei gegenüber der Horizontalen geneigten Röntgenuntersuchungsgerät mit in vertikaler Richtung verlaufender Röntgenstrahlung, die von einer in einer horizontalen Ebene befindlichen Kassette aufgezeichnet wird, z.B. für Röntgenaufnahmen am Krankenbett.

## Patentansprüche

1. Röntgenuntersuchungsgerät mit einem um eine erste Achse (3) schwenkbaren Patientenlagerungstisch (22), einem oberhalb der Tischplatte (4) des Patientenlagerungstisches angeordneten Zielgerät (6), das mittels einer Transporteinrichtung (10) parallel zur Tischplatte verschiebbar ist, sowie mit einem mit der Transporteinrichtung gekoppelten Röntgenstrahler (2),
dadurch gekennzeichnet, daß der Röntgenstrahler (2) um eine mit der Transporteinrichtung (10) oder einem damit fest verbundenen Teil (9) verbundene, zur Ebene der Tischplatte (4) und zur ersten Achse parallele zweite Achse (12) von einer auf das Zielgerät (6) ausgerichteten Position in eine Position oberhalb der Tischplatte (4) schwenkbar ist.

2. Röntgenuntersuchungsgerät nach Anspruch 1,
dadurch gekennzeichnet, daß die zweite (12) Achse im Abstand von einer zur Längsrichtung der Tischplatte senkrechten Ebene (13) angeordnet ist, die bei einer unterhalb der Tischplatte befindlichen Stellung des Röntgenstrahlers (2) dessen Fokus enthält.

3. Röntgenuntersuchungsgerät nach Anspruch 1,
dadurch gekennzeichnet, daß unter der Tischplatte (4) eine Laufrastereinrichtung (14) vorgesehen ist, die mit der Bewegung des Röntgenzielgeräts (6) in Längsrichtung koppelbar ist.

4. Röntgenuntersuchungsgerät nach Anspruch 1,
dadurch gekennzeichnet, daß der Röntgenstrahler (2) um eine zu der ersten und der zweiten (12) Achse parallele dritte Achse (18) schwenkbar ist, die durch den Fokus des Röntgenstrahlers (2) verläuft, daß der Röntgenstrahler (2) über eine Koppelstange (16) mit einem ortsfesten Punkt (17) am Röntgengerät gekoppelt ist und daß Mittel (19) zur motorischen Verschiebung des Röntgenstrahlers (2) in Längsrichtung des Patientenlagerungstisches (22) vorgesehen sind.

## Claims

1. An X-ray examination apparatus, including a patient table (22) which is pivotable about a first axis (3), a spot film device (6) which is arranged over the top (4) of the patient table and can be displaced parallel to the table top by means of a transport device (10), and also comprising an X-ray source (2) which is coupled to the transport device, characterized in that the X-ray source (2) is pivotable about a second axis (12) which is connected to the transport device (10), or to a part (9) rigidly connected thereto, and extends, parallel to the plane of the table top (4) and parallel to the first axis, from a position in line with the spot film device (6) to a position over the table top (4).

2. An X-ray examination apparatus as claimed in Claim 1, characterized in that the second axis (12) is situated at a distance from a plan (13), which extends perpendicularly to the longitudinal direction of the table top and contains the focus of the X-ray source (2) in a position underneath the table top.

3. An X-ray examination apparatus as claimed in Claim 1, characterized in that underneath the table top (4) there is provided a moving grid device (14) which can be coupled to the movement of the X-ray spot film device (6) in the longitudinal direction.

4. An X-ray examination apparatus as claimed in Claim 1, characterized in that the X-ray source (2) is pivotable about a third axis (18) which extends parallel to the first and the second axis (12) and through the focus of the X-ray source (2), that the X-ray source (2) is coupled to a stationary point (17) on the X-ray apparatus via a coupling rod (16), and that means (19) are provided for motor-driven displacement of the X-ray source (2) in the longitudinal direction of the patient table (22).

## Revendications

1. Appareil d'examen à rayons X avec une table d'examen de patient (22) pivotant autour d'un premier axe (3), un radiogoniomètre (6) disposé au-dessus du dessus (4) de la table d'examen du patient, lequel peut être déplacé parallèlement au dessus de la table à l'aide d'un dispositif de transport (10) ainsi qu'avec un émetteur de rayons X (2) couplé au dispositif de transport, caractérisé en ce que l'émetteur de rayons X (2) peut pivoter autour d'un deuxième axe (12) parallèle au premier axe et au plan du dessus de la table (4) et relié au dispositif de transport (10) ou à une pièce fixée à demeure avec celle-ci (9) d'une position orientée sur le radiogoniomètre (6) dans une position au-dessus du dessus de la table (4).

2. Appareil d'examen à rayons X selon la revendication 1, caractérisé en ce que le deuxième axe (12) est disposé à un intervalle d'un plan (13) perpendiculaire à la direction longitudinale du dessus de la table, plan qui, lorsque l'émetteur de rayons X se trouve sous le dessus de la table, contient son foyer.

3. Appareil d'examen à rayons X selon la revendication 1, caractérisé en ce qu'un dispositif de balayage (14) est prévu sous le dessus de la table (4) et peut être couplé avec le mouvement du radiogoniomètre X (6) en direction longitudinale.

4. Appareil d'examen à rayons X selon la revendication 1, caractérisé en ce que l'émetteur de rayons X (2) peut pivoter autour d'un troisième axe (18) parallèle à l'un des premier et deuxième (12) axes qui traverse le foyer de l'émetteur de rayons X (2), que l'émetteur de rayons X (2) est couplé par l'intermédiaire d'une tige d'accouplement (16) à un point stationnaire (17) sur l'appareil à rayons X et que des moyens (19) pour le décalage mécanique de l'émetteur de rayons X (2) dans la direction longitudinale de la table d'examen du patient (22) sont prévus.
